(19) Europäisches Patentamt
European Patent Office
Office européen des brevets

(11) Publication number: **0 487 735 B1**

(12) # EUROPEAN PATENT SPECIFICATION

(45) Date of publication of patent specification: **30.08.95**

(51) Int. Cl.6: **A61K 7/06**

(21) Application number: **91909720.4**

(22) Date of filing: **23.05.91**

(86) International application number:
**PCT/JP91/00692**

(87) International publication number:
**WO 91/18581 (12.12.91 91/28)**

(54) **EMULSIFIED HAIR CARE PREPARATION.**

(30) Priority: **30.05.90 JP 140624/90**

(43) Date of publication of application:
**03.06.92 Bulletin 92/23**

(45) Publication of the grant of the patent:
**30.08.95 Bulletin 95/35**

(84) Designated Contracting States:
**AT BE CH DE DK ES FR GB GR IT LI LU NL SE**

(56) References cited:
**EP-A- 0 285 364**
**JP-A- 0 222 212**
**JP-A- 0 259 510**
**JP-A-63 230 620**
**JP-A- 0 211 506**
**JP-A- 0 259 508**
**JP-A- 1 168 609**

**PATENT ABSTRACTS OF JAPAN, vol. 12, no. 462 (C-549)[3309], 5th December 1988; & JP-A-63 183 517 (SHISEIDO CO., LTD) 28-07-1988**

(73) Proprietor: **SUNSTAR KABUSHIKI KAISHA**
**3-1, Asahi-machi**
**Takatsuki-shi**
**Osaka 569 (JP)**

(72) Inventor: **YAMAMOTO, Kazushi, 2-1-3, Kyorit-sudori**
**Abeno-ku**
**Osaka-shi**
**Osaka 545 (JP)**
Inventor: **NANJO, Masashi, 3-20-12, Aomataninishi**
**Minoo-shi**
**Osaka 562 (JP)**
Inventor: **AWAMURA, Masaki, 2-14-3, Kotobuki-shi**
**Takatsuki-sh**
**Osaka 569 (JP)**

(74) Representative: **VOSSIUS & PARTNER**
**Postfach 86 07 67**
**D-81634 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 487 735 B1

## Description

The present invention relates to an emulsified hair cosmetic providing excellent easy combing and a feeling of smoothness to hair and protecting hair against heat and brushing.

Various hair cosmetics such as a hair treatment preparation, a hair rinse, etc. have been used to provide gloss and elasticity to hair, or to take care of injured hair. For example, in order to provide gloss to hair, a hair treating composition in which oily ingredients such as silicone oil, higher alcohol, liquid paraffin, ester oil, etc. are formulated have been used Further, in order to provide softness and antistatic effect to hair, a hair treating composition in which a cationic surfactant or a cationic polymer is formulated have been used. Furthermore, in order to protect hair against heat of drier, etc., a hair treating composition in which a water-soluble polymer is formulated have been used.

However, when a hair treating composition in which the above oily ingredients are formulated is applied to hair, it provides a feeling of extraneous matter such as sticking . The hair treating composition, itself also have sticking, therefore the physical resistance against combing or brushing becomes large. Further, a hair treating composition in which a cationic surfactant, a cationic high polymer is formulated electrically adsorbs only at the part of a surface of hair, charged negative, so that the adsorptivity is not enough and the homogeneity and the combing are not satisfactory. Furthermore, a hair treating composition itself in which a water-soluble polymer is formulated have a remarkable sticking and adhesion, therefore after treatment it provides a feeling of extraneous matter such as stiffness and the combing is not satisfactory.

In order to overcome these problems, a hair care product using a silicone rubber is recently provided (Japanese Patent Laid Open Application No. 313714/1988, Japanese Patent Laid Open Application No. 168609/1989). Further, there is a demand for a product having more excellent properties.

According to the present invention, there is provided an emulsified hair cosmetic which comprises

(i) a dimethylsilicone rubber of the formula:

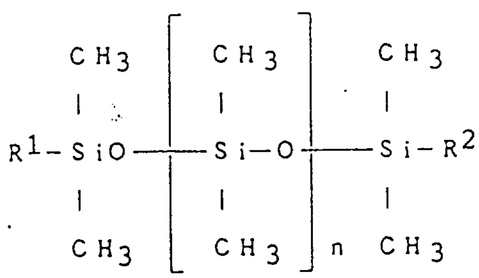 [I]

wherein $R^1$ and $R^2$ are methyl or hydroxy; and n is an integer of 4000 to 9000;

(ii) a dimethylsilicone oil having the viscosity of 10 to 100000 $mm^2/s$ (centistokes) at 25 °C;

(iii) one or more of polyhydric alcohol selected from the group consisting of propylene glycol, 1,3-butylene glycol and glycerin; and

(iv) one or more of nonionic surfactant selected from the group consisting of polyoxyethylene oleyl ether, polyoxyethylene cetyl ether, ethylene glycol stearate, diethylene glycol stearate, polyethylene glycol stearate, polyethylene glycol distearate and glyceryl stearate, the amount of (i) being 1/20 to 1/3 of the amount of (ii), the total amount of (iv) being not 7-fold more than the amount of (i) and being 1/2 to 2 of the amount of (iii).

The emulsified hair cosmetic of the present invention provides excellent easy combing and smooth feeling to hair and protects hair against heat and brushing.

Representative examples of dimethyl silicone rubber shown by the formula [I] which are used in the hair treating compositions of the present invention include Toshiba Silicone TSE-200® and TSE-200® manufactured by Toshiba Silicone Co., Ltd., Japan and the like, and one or more of them can be formulated in the emulsified hair cosmetic of the present invention in an amount of 0.01 to 10% by weight, preferably 0.5 to 2.0% by weight based on the total weight of the cosmetic. When the amount is less than 0.01% by weight, the effect for providing easy combing and a feeling of smoothness to hair becomes insufficient and, when the amount is more than 10% by weight, viscosity of the dimethyl silicone rubber itself increases and it becomes difficult to emulsify.

A dimethyl silicone oil of (ii) is known as so-called nonvolatile dimethyl silicone oil having the viscosity of 10 to 100000 $mm^2/s$ (centistokes) at 25 °C. When viscosity is less than 10 $mm^2/s$ (centistokes), the dimethyl silicone oil is easy to volatilize and durability of the effect of the hair cosmetic becomes inferior,

and when viscosity is more than 100000 centistokes, the hair cosmetic becomes too viscous and a feeling of use becomes bad. Such the dimethyl silicone oil can be formulated in the emulsified hair cosmetic in an amount of 3 to 20-fold weight of silicone rubber (i). When the amount is 3-fold less than that of silicone rubber, viscosity of hair cosmetic increases and a feeling of use becomes bad. And when the amount is 20-fold more than the amount of silicone rubber, the properties of the hair cosmetic is adversely effected and the effect for providing easy combing becomes bad.

In the hair cosmetic of the present invention, the total amount of nonionic surfactant (iv) is not 7-fold more than the amount of silicone rubber (i), and is 1/2 to 2 of the amount of polyalcohol (iii). When the total amount of (iv) is 7-fold more than the amount of silicone rubber, the emulsification is stabilized but usability of the emulsion is adversely effected. Further, when the total amount of nonionic surfactant is out of the range of 1/2-fold to 2-fold amount of polyalcohol of (iii), the emulsion becomes unstable, and liable to separate with the passage of time.

A hair cosmetic of the present invention can be prepared in a form of milky lotion, blow agent, hair rinse, hair treatment or hair mousse by mixing desired ingredients and emulsifying them. Further, oily ingredients (such as cyclic silicone, triglyceride, ester oil, wax, etc.), coloring agents, perfumery, pH adjustors (such as phosphoric acid, citric acid, etc.), humectants (such as pyrrolidone carboxylate, lactic acid, etc.), solvents (such as water, ethanol, etc.), antistatic agents (such as cationic surfactant, etc.) can be formulated in the emulsified hair cosmetic of the present invention so far as they do not deteriorate the properties of the composition.

The following Examples and Comparative Examples further illustrate the present invention in detail. All the %'s in Examples and Comparative Examples are by weight.

Examples 1 to 6 and Comparative Examples 1 to 10

Each milky lotion like hair cosmetic was prepared by mixing the ingredients described in Table 1 and thereafter stirring according to a conventional method. The following items were evaluated by the use of these hair cosmetics. The results are also shown in Table 1.

Evaluation

Evaluation of a test sample was carried out according to the following method.

(1) Sticking and gloss immediately after application

These were evaluated by organoleptic test of practical use involving five professional panelists (female of the twenties).

0: Gloss, no sticking immediately after application
X: Sticking, no gloss immediately after application

(2) Resistance against combing after drying

A human hair (2g) treated with each test treating composition and dried was attached to a rheometer (Fudo Kogyo Co., Ltd., Japan). Maximum drag applied to the rheometer upon combing was measured and resistance against combing was calculated from the following formula:

$$\text{Resistance against combing (\%)} = \frac{\text{Resistance against combing after treatment}}{\text{Resistance against combing before treatment}} \times 100$$

(3) The number of hair cut

The number of hair cut after 7200 times of combing (a bundle of hair weighed about 2g: 119 non-treated hairs)

(4) Stability

After being allowed to stand at 40 °C for one month, separation (or creaming ) was evaluated according to the following criteria.

0: Stable
X: Creaming or separation

(5) Overall evaluation

0: No sticking, easy combing and stable
X: Sticking, bad combing or unstable

Table 1

| | Ingredients | Amount (% by weight) Examples 1 | 2 | 3 | 4 | 5 | 6 | Comparative Examples 1 | 2 | 3 | 4 | 5 | 6 | 7 | 8 | 9 | 10 |
|---|---|---|---|---|---|---|---|---|---|---|---|---|---|---|---|---|---|
| i | Dimethyl silicone rubber ($R^1$ and $R^2$=$CH_3$) | 1.0 | - | 0.8 | - | 0.2 | 1.5 | - | 1.0 | 0.5 | 1.0 | 1.0 | 0.5 | 0.5 | 1.0 | 0.5 | 0.5 |
| | Dimethyl silicone rubber ($R^1$ and $R^2$=OH) | - | 1.0 | - | 0.5 | - | - | - | - | - | - | - | - | - | - | - | - |
| ii | Dimethyl silicone oil 2 $mm^2/s$ (2cs) | - | - | - | - | - | - | - | - | - | 5.0 | - | - | - | - | - | - |
| | Dimethyl silicone oil 10 $mm^2/s$ (10cs) | - | 5.0 | - | - | 2.0 | 10.0 | - | - | - | - | - | - | - | - | - | - |
| | Dimethyl silicone oil 100 $mm^2/s$ (100cs) | 5.0 | - | - | 5.0 | 2.0 | - | 5.0 | 2.5 | 12.0 | - | - | 5.0 | 5.0 | 5.0 | 5.0 | 5.0 |
| | Dimethyl silicone oil 100000 $mm^2/s$ (100000cs) | - | - | 5.0 | - | - | - | - | - | - | - | - | - | - | - | - | - |
| | Dimethyl silicone oil 1000000 $mm^2/s$ (1000000cs) | - | - | - | - | - | - | - | - | - | - | 5.0 | - | - | - | - | - |
| iii | Propylene glycol | 1.0 | - | - | 1.0 | 0.5 | - | 1.0 | - | - | 1.0 | - | - | 5.0 | 1.0 | 1.0 | 1.0 |
| | Glycerin | - | 1.0 | - | - | - | 0.5 | - | 1.0 | - | - | 1.0 | - | - | - | - | - |
| | 1,3-Butylene glycol | - | - | 1.0 | - | 0.5 | 0.5 | - | - | 1.0 | - | - | - | - | - | - | - |
| iv | Polyoxyethylene oleyl ether (5EO) | 1.2 | - | - | 0.5 | - | 0.5 | 1.2 | 1.2 | 1.2 | 1.2 | 1.2 | 1.2 | 1.2 | - | 2.0 | 1.5 |
| | Polyoxyethylene cethyl ether (10EO) | - | 0.3 | - | 0.2 | - | 0.8 | - | - | - | - | - | - | - | - | 2.0 | 1.0 |
| | Glyceryl stearate | - | 0.8 | - | 0.3 | 0.2 | - | - | - | - | - | - | - | - | - | - | - |
| | Polyoxyethylene stearate (20EO) | - | - | 1.2 | - | 1.0 | - | - | - | - | - | - | - | - | - | - | - |
| | Ethanol | 10.0 | 10.0 | 10.0 | - | 5.0 | - | 10.0 | 10.0 | 10.0 | 10.0 | 10.0 | 10.0 | 10.0 | 10.0 | 10 0 | 10.0 |
| | Isopropyl palmitate | 1.0 | - | - | 0.5 | - | - | 1.0 | - | - | - | - | - | - | - | - | - |
| | Tween 80 | - | - | - | - | - | - | - | - | - | - | - | - | - | 1.2 | - | - |
| | Sodium lauryl sulphate | - | - | - | 0.1 | - | - | - | - | - | - | - | - | - | - | - | - |
| | Water | Remainder | | | | | | | | | | | | | | | |

Contd.

| Result | Examples | | | | | | Comparative Examples | | | | | | | | | |
|---|---|---|---|---|---|---|---|---|---|---|---|---|---|---|---|---|
| | 1 | 2 | 3 | 4 | 5 | 6 | 1 | 2 | 3 | 4 | 5 | 6 | 7 | 8 | 9 | 10 |
| Sticking immediately after application | O | O | O | O | O | O | O | X | X | O | X | - | - | - | X | X |
| Gloss of hair | O | O | O | O | O | O | O | X | O | X | O | - | - | - | X | X |
| Resistance against combing (%) | 62.3 | 55.2 | 59.3 | 57.8 | 63.0 | 55.0 | 101.1 | 65.2 | 93.1 | 60.3 | 83.2 | - | - | - | 82.3 | 90.3 |
| Resistance against combing (after drying by drier, %) | 65.7 | 58.3 | 60.1 | 58.9 | 63.2 | 58.2 | 102.5 | 69.3 | 95.7 | 78.3 | 87.2 | - | - | - | 85.5 | 89.5 |
| The number of hair cut | 5 | 2 | 7 | 3 | 3 | 5 | 105 | 9 | 92 | 9 | 70 | - | - | - | 62 | 97 |
| Stability | O | O | O | O | O | O | O | O | O | O | O | X | X | X | O | O |
| Overall evaluation | O | O | O | O | O | O | X | X | X | X | X | X | X | X | X | X |

Example 7 (a blow-wave preparation)

| Ingredient A | Amount (%) |
|---|---|
| 1) Propylene glycol<br>2) Polyoxyethylene oleyl ether (5 EO)<br>3) Polyoxyethylene stearate (2 EO)<br>4) Dimethyl silicone rubber (TSE 200A®)<br>5) Polyoxyethylene lauryl ether sulfuric acid sodium salt<br>6) Dimethyl silicone oil (100 $mm^2/s$ (cs)) | 0.8<br>0.5<br>0.2<br>0.8<br>0.1<br>8.0 |
| Ingredient B | Amount (%) |
| 7) Water | remainder |
| Ingredient C | Amount (%) |
| 8) 95% Ethanol | 20 0 |
| Ingredient D | Amount (%) |
| 9) Perfume | 0.1 |

To the ingredient A heated at 60 °C added the ingredient B heated at 60 °C and they were stirred to mix. Then, ingredients C and D were added while cooling. Alternatively, a mixture of surfactant and water-soluble polyalcohol may be prepared at first, and then an oily phase may be added.

Example 8 (a milky lotion type)

| Ingredient A | Amount(%) |
|---|---|
| 1) 1,3-Butylene glycol<br>2) Polyoxyethylene cetyl ether (5 EO)<br>3) Dimethyl silicone rubber (TSE 200®)<br>4) Dimethyl silicone oil (20 $mm^2/s$ (cs)) | 1.8<br>1.2<br>1.0<br>19.0 |
| Ingredient B | Amount (%) |
| 5) Carbopol 941<br>6) Water | 0.3<br>remainder |
| Ingredient C | Amount (%) |
| 7) Triethanolamine<br>8) Water | 0.3<br>2.7 |
| Ingredient D | Amount (%) |
| 9) Perfume | 0.1 |

Ingredient B were mixed and kept at 80 °C. Separately, ingredient A were mixed and kept at 70 °C, and this was added to the above-described ingredient B, and these were stirred into uniformity. Thereafter, ingredient C and ingredient D were added to prepare a hair milky lotion.

Example 9 (a hair rinse)

| Ingredient A | Amount (%) |
|---|---|
| 1) Stearyltrimethylammonium chloride | 2.0 |
| 2) Dimethyl silicone rubber (TSE200A®) | 2.0 |
| 3) Dimethyl silicone oil (500 $mm^2/s$ (cs)) | 8.0 |
| 4) Self-emulsifiable glyceryl monostearate | 1.0 |
| 5) Ethylene glycol monostearate | 1.0 |
| **Ingredient B** | **Amount (%)** |
| 6) Glycerin | 2.0 |
| 7) Preservative | trace |
| 8) Coloring agent | trace |
| 9) Water | remainder |
| **Ingredient C** | **Amount (%)** |
| 10) Perfume | 1.0 |

Ingredient B were mixed and kept at 75 °C, and to this added ingredient A which had been separately mixed and kept at 75 °C. To this added ingredient C while stirring and cooling to prepare a hair rinse.

Example 10 (a hair treatment type)

| Ingredient A | Amount (%) |
|---|---|
| 1) Dimethylbenzylammonium chloride | 3.0 |
| 2) Dimethyl silicone rubber (TSE200®) | 1.5 |
| 3) Dimethyl silicone oil (10 $mm^2/s$ (cs)) | 10.0 |
| 4) Lanolin | 1.0 |
| 5) Squalane | 2.0 |
| 6) Self-emulsifiable glyceryl monostearate | 3.0 |
| 7) Ethylene glycol monostearate | 5.0 |
| 8) Cetyl alcohol | 0.5 |
| **Ingredient B** | **Amount (%)** |
| 9) Propylene glycol | 5.0 |
| 10) Sodium hyaluronate | 0.001 |
| 11) Preservative | trace |
| 12) Water | remainder |
| **Ingredient C** | **Amount (%)** |
| 13) Perfume | trace |

Ingredient B were mixed and kept at 75 °C, and to this added ingredient A which had been separately mixed and kept at 75 °C. To this added ingredient C while stirring and cooling to prepare a hair treatment type composition.

Example 11 (a hair mousse)

| Ingredient A | Amount (%) |
|---|---|
| 1) Propylene glycol<br>2) Polyoxyethylene oleyl ether (5 EO)<br>3) Dimethyl silicone rubber (TSE200A®)<br>4) Dimethyl silicone oil (100 $mm^2/s$ (cs)) | 1.0<br>1.0<br>0.3<br>4.0 |
| Ingredient B | Amount (%) |
| 5) Witch-hazel extract<br>6) Polyvinyl pyrrolidone<br>7) Water | 0.01<br>0.8<br>remainder |
| Ingredient C | Amount (%) |
| 8) 95% Ethanol<br>9) Perfume | 10.0<br>0.1 |

To the ingredient A heated at 60 °C added the ingredient B heated at 60 °C, and these were stirred to mix. Then, to this added the ingredient C while cooling to prepare an undiluted solution. 90 Parts of this undiluted solution and 10 parts of propellant were filled into an aerosol container to obtain a hair mousse.

## Claims

**1.** An emulsified hair cosmetic which comprises
(i) 0.01 - 10% by weight of a dimethylsilicone rubber of the formula:

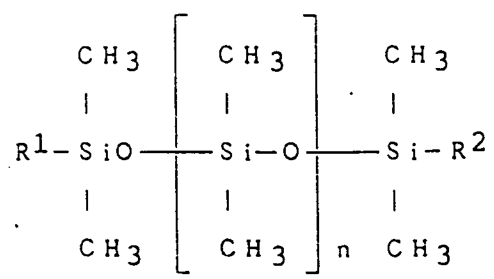

[ I ]

wherein $R^1$and $R^2$ are methyl or hydroxyl; and n is an integer of 4000 to 9000;
(ii) a dimethylsilicone oil having the viscosity of 10 to 100000 $mm^2/s$ (centistokes) at 25 °C;
(iii) one or more of polyhydric alcohol selected from the group consisting of propylene glycol, 1,3-butylene glycol and glycerin; and
(iv) one or more of nonionic surfactant selected from the group consisting of polyoxyethylene oleyl ether, Polyoxyethylene cetyl ether, ethylene glycol stearate, diethylene glycol stearate, polyethylene glycol stearate, polyethylene glycol distearate and glyceryl stearate, the amount of (i) being 1/20 to 1/3 of the amount of (ii), the total amount of (iv) being not 7-fold more than the amount of (i) and being 1/2 to 2 of the amount of (iii).

## Patentansprüche

1. Emulgiertes Haarpflegemittel, das umfaßt:
(i) 0,01 - 10 Gewichts-% eines Dimethylsilikongummis der Formel:

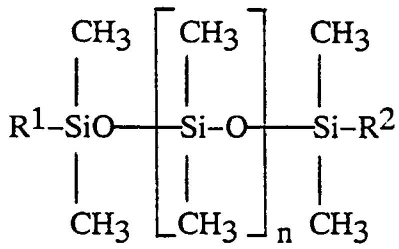

[I],

worin $R^1$ und $R^2$ Methyl oder Hydroxyl sind; und n eine ganze Zahl von 4000 bis zu 9000 ist;
(ii) Dimethylsilikonöl, das die Viskosität von 10 bis zu 100.000 $mm^2/s$ (Zentistokes) bei 25 °C aufweist;
(iii) einen oder mehrere mehrwertige Alkohole, die ausgewählt sind aus der Gruppe, die aus Propylenglykol, 1,3-Butylenglykol und Glycerin besteht; und
(iv) ein oder mehrere nichtionische oberflächenaktive Mittel, die ausgewählt sind aus der Gruppe, die aus Polyoxyethylenoleylether, Polyoxyethylencetylether, Ethylenglykolstearat, Diethylenglykolstearat, Polyethylenglykolstearat, Polyethylenglykoldistearat und Glycerylstearat besteht, wobei die Menge von (i) 1/20 bis 1/3 der Menge von (ii) beträgt, die Gesamtmenge von (iv) nicht mehr als siebenfach die Menge von (i) beträgt und die halbe bis doppelte Menge von (iii) beträgt.

## Revendications

1. Produit de beauté émulsifié pour cheveux qui comprend :
(i) de 0,01 à 10 % en masse d'un caoutchouc de diméthylsilicone de formule :

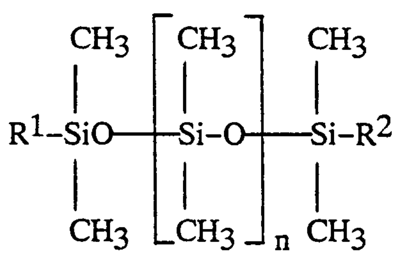

[I]

dans laquelle $R^1$ et $R^2$ représentent le groupement méthyle ou hydroxyle et n est un nombre entier compris entre 4 000 et 9 000 ;
(ii) une huile de diméthylsilicone ayant une viscosité comprise entre 10 et 100 000 $mm^2/s$ (centistokes) à 25 °C ;
(iii) un ou plusieurs polyalcools choisis dans le groupe constitué du propylèneglycol, du 1,3-butylèneglycol et du glycérol ; et
(iv) un ou plusieurs tensioactifs non ioniques choisis dans le groupe constitué de l'oxyde de polyoxyéthylène et d'oléyle, de l'oxyde de polyoxyéthylène et de cétyle, du stéarate d'éthylèneglycol, du stéarate de diéthylèneglycol, du stéarate de polyéthylèneglycol, du distéarate de polyéthylèneglycol et du stéarate de glycéryle, la quantité de (i) étant de 1/20 à 1/3 de la quantité de (ii), la quantité totale de (iv) n'étant pas 7 fois supérieure à la quantité de (i) et étant de 1/2 à 2 fois la quantité de (iii).